# EUROPEAN PATENT SPECIFICATION

(11) **EP 4 496 787 B1**
(45) Date of publication and mention of the grant of the patent: **28.05.2025**
(21) Application number: 23706859.8
(22) Date of filing: 09.02.2023
(51) Int. Cl.: C07C 323/41, C09J 177/00, C08G 69/00

(54) **THIOL-OXAMIDE COMPOUNDS AND COMPOSITIONS**
THIOL-OXAMID-VERBINDUNGEN UND ZUSAMMENSETZUNGEN
COMPOSÉS DE THIOL-OXAMIDE ET COMPOSITIONS

(30) Priority: 22.03.2022 US 202263269750 P
(43) Date of publication of application: 29.01.2025
(73) Proprietor: 3M Innovative Properties Company, Saint Paul, Minnesota 55133-3427 (US)
(72) Inventor: SORENSON, Gregory P., Saint Paul, Minnesota 55133-3427 (US); WHITE, Kolby L., Saint Paul, Minnesota 55133-3427 (US)
(74) Representative: Bergen, Katja
(86) International application number: PCT/IB2023/051185
(87) International publication number: WO 2023/180824

(56) References cited:
- WO-A2-2010/011714
- US-A- 6 153 719

## Description

### Background

The reaction between thiol-containing compounds and oxirane-containing compounds has been extensively leveraged in the development of adhesives and sealants. These reactions are particularly attractive because of their high conversion efficiency and fast reaction kinetics. Reactions between multifunctional thiols (i.e., compounds with multiple thiol groups) and multifunctional epoxies (i.e., compounds with multiple oxirane groups) have been successfully used in the preparation of fast curing structural adhesives. Adhesives that leverage these chemistries, however, often have polymer networks with softening temperatures below 35 degrees Celsius. Such low softening temperatures can limit the scope of these adhesives to applications with low process temperatures and low use temperatures. One common method to overcome this limitation includes using highly functional monomers to generate tightly crosslinked networks. While such a strategy can be useful for increasing the softening temperature of the polymer, the product is often brittle.

An additional limitation to the use of thiol-containing compounds as curable materials is the lack of diversity in commercially available compounds. Often multifunctional thiols that are required to build polymer networks are derived from esters of thioglycolic or thiopropionic acid. Because of the ester groups in the backbone of the polymers derived from these materials, however, the polymers tend to be sensitive to hydrolysis and the physical properties of the polymers can decline during aging.

US 6153719A discloses thiol-containing compounds for use in polymerization.

### Summary

A thiol-containing compound having at least two terminal groups of formula

-NH-CO-CO-NH-R¹-SH

where R¹ is an alkylene, reaction mixtures containing the thiol-containing compound and an epoxy resin, and compositions containing a polymerized product of the reaction mixture are provided. The compositions that contain the polymerized product of the reaction mixture can be used, for example, as an adhesive or sealant.

In a first aspect, a thiol-containing compound having at least two terminal groups of formula -NH-CO-CO-NH-R¹-SH is provided. Group R¹ is an alkylene.

In a second aspect, a reaction mixture is provided that contains (a) an oxamido-containing compound having at least two terminal groups of formula -NH-CO-CO-NH-R¹-SH and (b) an epoxy resin. Group R¹ is an alkylene group.

In a third aspect, a composition is provided that contains a polymerized product of a reaction mixture described above in the second aspect. The polymerized product can be either a thermoplastic or thermoset material.

The terms "a", "an", and "the" are used interchangeably with "at least one" to mean one or more of the elements being described.

The term "and/or" means either or both. For example, the expression X and/or Y means X, Y, or a combination thereof (both X and Y).

The term "alkylene" refers to a divalent group that is a radical of an alkane and includes groups that are linear, branched, cyclic, bicyclic, or a combination thereof. Unless otherwise indicated, the alkylene group typically contains 1 to 30 carbon atoms. In some embodiments, the alkylene group contains 1 to 20 carbon atoms, 1 to 10 carbon atoms, 1 to 6 carbon atoms, 1 to 4 carbon atoms, or 1 to 3 carbon atoms. Branched and cyclic alkylene groups have at least 3 carbon atoms and bicyclic alkylene groups typically have at least 7 carbon atoms. Example alkylene groups include, but are not limited to, methylene, ethyl, n-propylene, n-butylene, n-pentylene, isobutylene, t-butylene, isopropylene, n-octylene, n-heptylene, ethylhexylene, cyclopentylene, cyclohexylene, cycloheptylene, adamantylene, norbomylene, and the like.

The term "heteroalkylene" refers to alkylene where at least one carbon atom between two other carbon atoms is replaced with a heteroatom. The heteroatom is typically oxygen (-O-), nitrogen (-NR^{a}- where R^{a} is hydrogen or alkyl), or sulfur (-S-). The heteroalkylene often has one or more oxygen heteroatoms and/or one or more sulfur atoms. If there are more than one heteroatom, they are typically separated by at least one carbon atom. For example, the oxygen heteroatoms are not peroxides.

The term "(hetero)alkylene" refers to an alkylene, heteroalkylene, or both.

The term "ether group" refers to a group having an oxygen atom between two alkylene groups.

The term "polyether group" is a heteroalkylene having multiple oxygen heteroatoms. The heteroalkylene often contains multiple ethylene groups or propylene groups separated by oxygen heteroatoms.

The term "oxamido" refers to a divalent group of formula -NH-(C=O)-(C=O)-NH-, which can also be written -NH-CO-CO-NH-.

The term "room temperature" refers to a temperature ranging from 20 to 25 degrees Celsius or 22 to 25 degrees Celsius.

The phrase "in a range of", "ranging from", or a similar phrase refers to all values within the stated range plus the endpoints of the range.

Dashes on either side of groups such as -O- and -NH- indicate that these groups are divalent. A dash on a single side of a group such as -CH₃ indicates that this group is monovalent.

As used herein, any statement of a range includes the endpoint of the range and all suitable values within the range (e.g., 1 to 5 includes 1, 1.5, 2, 2.75, 3, 3.80, 4, 5, etc.).

### Detailed Description

A thiol-containing compound is provided that has a plurality of oxamido groups as well as a plurality of thiol groups. The thiol-containing compounds can be combined with an epoxy resin having a plurality of oxirane groups to form a reaction mixture for a polymerized composition that can have either thermoplastic or thermoset characteristics. The oxamido groups in the thiol-containing compounds advantageously can increase the hydrogen bonding in the polymerized composition compared to many conventional thiol-containing compounds. This increased hydrogen bonding can result in the polymerized product having an elevated glass transition temperature as well as other desirable properties such as good thermal resistance and toughness.

### Thiol-containing compound

The thiol-containing compound has a plurality of oxamido groups as well as a plurality of thiol groups. This compound typically has at least two groups of formula -NH-CO-CO-NH-R¹-SH where R¹ is an alkylene. For example, the thiol-containing compound can have at least 2, at least 3, at least 4, or at least 5 and up to 10, up to 8, up to 6, or up to 4 groups of formula -NH-CO-CO-NH-R¹-SH.

Suitable alkylene R¹ groups often have 2 to 10 carbon atoms but larger alkylene groups can be used. The alkylene often has at least 2, at least 3, at least 4, or at least 5 carbon atoms and up to 10, up to 8, up to 6, or up to 4 carbon atoms. The alkylene groups can be linear, branched, cyclic, or a combination thereof.

The thiol-containing compound is often of Formula (I).

R²-(NH-CO-CO-[NH-R³-NH-CO-CO]ₓ-NH-R¹-SH)ₙ (I)

In Formula (I), the group R¹ is the same as described above. Group R² is an n-valent radical (i.e., a radical with a valency equal to the variable n) of (hetero)hydrocarbon where the variable n is equal to at least 2. Group R² is typically a residual of a polyamine compound of formula R²(NH₂)ₙ minus the amino groups (i.e., the number of amino groups is equal to n). Group R³ is a (hetero)alkylene. Group R³ is often a residual of a diamine compound of formula R³(NH₂)₂ minus the two amino groups. The variable x is an integer in a range of 0 to 5.

Group R² is an n-valent radical that is a residual of a polyamine compound minus the two or more amino groups. The polyamine compound is not a silicone-based amine compound such as, for example, a polydimethylsiloxane having two or more amino groups. The residue is usually an n-valent radical of a (hetero)hydrocarbon. Some R² groups are an n-valent radical of hydrocarbon. Such R² groups are often an alkane-diyl (i.e., an alkylene) or an alkane-triyl. Other hydrocarbon R² groups have an aromatic ring with two attached alkylene groups; these groups are often alkylkene-arylene-alkylene groups. Still other R² groups are n-valent radicals of a heterohydrocarbon having -O-, -NH-, or -S- heteroatoms. These R² groups have a plurality of groups -R^{b}-X-R^{b}- where each R^{b} is ethylene, propylene, or tetramethylene, and each X is -O-, -NH-, or -S-. In many embodiments, the heterohydrocarbon is a polyether group (X is -O-). That is, R² is an n-valent radical of a polyether.

Suitable n-valent radicals of a hydrocarbon can have any suitable number of carbon atoms but often has 3 to 60 carbon atoms. In some embodiments, R² is a n-valent radical of an alkane that has at least 3, at least 4, at least 6, or at least 10, at least 20, at least 30, or at least 40 and up to 60, up to 50, up to 40, up to 30, up to 20, or up to 10 carbon atoms.

Suitable R² groups that are n-valent radicals of a heterohydrocarbon can have any suitable number of carbon atoms and heteroatoms. In other embodiment, R² is a n-valent radical of a polyether having at least 12 carbon atoms and at least 2 oxygen atoms. The polyether is often polyethylene oxide, polypropylene oxide, poly(tetramethylene oxide), or a copolymer thereof. The total number of carbon atoms is often up to 300 and the total number of oxygen atoms is often up to 100. For example, the polyether can have at least 12, at least 16, at least 20, at least 40, at least 60, at least 80, or at least 100 and up to 300, up to 275, up to 250, up to 225, up to 200, up to 175, up to 150, up to 125, up to 100, up to 75, or up to 50 carbon atoms. Further, the polyether can have at least 2, at least 4, at least 10, at least 20, at least 30, at least 40, or at least 50 and up to 100, up to 80, up to 70, up to 60, up to 50, up to 40, up to 30, or up to 20 oxygen atoms.

The variable n in Formula (I) is an integer equal to at least 2 and is often in a range of 2 to 10. For example, the variable n is at least 2, at least 3, or at least 4 and up to 10, up to 8, up to 6, or up to 4. In some embodiments, n is equal to 2, 3, or 4. The variable x is an integer in a range of 0 to 5. In some embodiments, x is equal to 0, 1, 2, or 3.

The compound R²(NH₂)ₙ is not a silicone-based amine compound. More particularly, group R² is not a polydiorganosilixane segment (e.g., polydimethylsiloxane segment).

Group R³ in Formula (I) is a (hetero)alkylene and is the residue of a diamine compound minus two amino group. Suitable alkylene R³ groups often have 2 to 60 carbon atoms. In some embodiments, R³ is an alkylene that has at least 3, at least 4, at least 6, or at least 10, at least 20, at least 30, or at least 40 and up to 60, up to 50, up to 40, up to 30, up to 20, or up to 10 carbon atoms. Suitable heteroalkylene R³ groups can have any suitable number of carbon atoms and heteroatoms. In many embodiments, the heteroatoms are oxygen. For example, R³ can be a heteroalkylene having at least 12 carbon atoms and at least 2 oxygen atoms. This R³ is a divalent radical of a polyether such as polyethylene oxide, polypropylene oxide, poly(tetramethylene oxide), or a copolymer thereof. The total number of carbon atoms is often up to 300 and the total number of oxygen atoms is often up to 100. For example, the polyether can have at least 12, at least 16, at least 20, at least 40, at least 60, at least 80, or at least 100 and up to 300, up to 275, up to 250, up to 225, up to 200, up to 175, up to 150, up to 125, up to 100, up to 75, or up to 50 carbon atoms. Further, the polyether can have at least 2, at least 4, at least 10, at least 20, at least 30, at least 40, or at least 50 and up to 100, up to 80, up to 70, up to 60, up to 50, up to 40, up to 30, or up to 20 oxygen atoms.

The variable x in Formula (I) is an integer in a range of 0 to 5. In some embodiments, x is equal to 0, 1, 2, or 3. In some embodiments, x is equal to 0 and Formula (I) is of Formula (I-1).

R²-(NH-CO-CO-NH-R¹-SH)ₙ (I-1)

In Formula (I-1), the groups R¹ and R² as well as the variable n are the same as described above for Formula (I).

The compound of Formula (I-1) can be formed, for example, according to Reaction Scheme A. 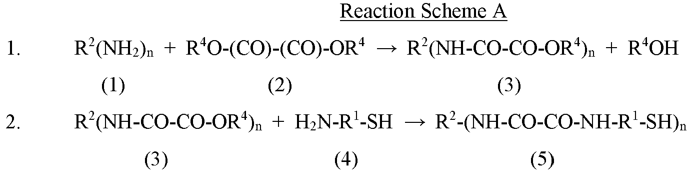 In a first reaction, the polyamine of formula R²(NH₂)ₙ (compound 1) is reacted with the oxalate compound (compound 2) of formula R⁴O-(CO)-(CO)-OR⁴ to form compound 3 of formula R²(NH-CO-CO-OR⁴)ₙ. The product of the first reaction (compound 3) is then reacted with a compound formula H₂N-R¹-SH (compound 4) having both an amino group and a thiol group. The product of the second reaction is compound (5), which is a compound of Formula (I-1).

Suitable examples of the polyamine R²(NH₂)ₙ (compound 1) include, but are not limited to, various difunctional amine-terminated polyethers that are available under the trade designation JEFFAMINE D, and JEFFAMINE ED from Huntsman Corporation (The Woodlands, TX, USA). These polyamines typically have two amino groups bonded to tertiary carbon atoms. Examples include JEFFAMINE D-230, D-200, D-400, ED-600, ED-900, and ED-2003 where the number is indicative of the molecular weight (e.g., weight average molecular weight). Other polyether amines are available from Huntsman Corporation under the trade designation JEFFAMINE THF such as JEFFAMINE THF-100 with a molecular weight of about 1000 grams/mole and JEFFAMINE THF-170 with a molecular weight of about 1700 grams/mole (e.g., weight average molecular weight). Still other polyamines include those commercially available from EVONIK (Essen, Germany) under the trade designation VERSALINK P such as VERSALINK P-650 and VERSALINK P-1000.

The polyamine compounds of formula R²(NH₂)ₙ can have more than two amino groups. In some embodiments, the polyamine is a trifunctional amine-terminated polyether such as those that are available under the trade designation JEFFAMINE T from Huntsman Corporation. These include JEFFAMINE T-403, T-3000, and T-5000 where the number is indicative of the molecular weight (weight average molecular weight for a polymer). Several branched polyethylenimine compounds are commercially available from Sigma-Aldrich (St. Louis, MO, USA) and Polysciences (Warrington, PA, USA).

Still other polyamine compounds of formula R²(NH₂)ₙ (compound 1) include propylene diamine, butylene diamine, 2-methylpentane-1,5-diamine, 1,6-hexanediamine, 1,8-octanediamine, 1,10-decanediamine, 1,12-dodecanediamine, 4,7,10-trioxa-1,13-tridecanediamine (TTD), bis(aminomethyl)cyclohexane, isophorone diamine, 4,4'-methylenebis(cyclohexylamine), 2,2,4-trimethyl-1,6-diaminohexane, 2,4,4-trimethyl-1,6-diaminohexane, octahydro-4,7-methano-1H-indenedimethylamine (available under the trade designation TCD DIAMINE from Oxea, Dallas, Tex.), bis(aminoethyl)benzene, 3,6-dioxaoctane-1,8-diamine, xylene diamine, diethylene triamine, triethylene tetramine, bis(aminomethyl)norbornane, dipropylene triamine, tetraethylene pentaamine, and hexaethylene heptamine.

In Reaction Scheme A, the polyamine (compound 1) is reacted with an oxalate compound (compound 2) of formula R⁴O-(CO)-(CO)-OR⁴. The group R⁴ is a hydrocarbyl that is usually an alkyl, aryl, aralkyl, or alkaryl. Suitable alkyl groups often have 1 to 10 carbon atoms, 1 to 6 carbon atoms, or 1 to 4 carbon atoms. Suitable aryl groups typically have 6 to 10 carbon atoms. The aryl is often phenyl. Suitable aralkyl groups often have an aryl group with 6 to 10 carbon atoms (e.g., phenyl) and an alkylene group with 1 to 10 carbon atoms. The aralkyl is often benzyl. Suitable alkaryl groups often have an arylene group with 6 to 10 carbon atoms (e.g., phenylene) and an alkyl group with 1 to 10 carbon atoms. The alkaryl is often tolyl.

Oxalate compounds can be prepared, for example, by reacting an alcohol of formula R⁴-OH with oxalyl dichloride. Oxalate compounds that are commercially available include, but are not limited to, dimethyl oxalate, diethyl oxalate, di-n-butyl oxalate, di-*tert*-butyl oxalate, diisopropyl oxalate, dipropyl oxalate, dipentyl oxalate, *tert*-butyl ethyl oxalate, *tert*-butyl methyl oxalate, bis(4-methylbenzyl) oxalate, isobutyl octan-2-yl oxalate, dibenzyl oxalate, and bis(phenyl) oxalate.

The reaction product of the polyamine compound of formula R²(NH₂)ₙ with the oxalate compound (compound 2) in Reaction Scheme A is a compound of formula R²(NH-CO-CO-OR ⁴)ₙ (compound 3). The groups R² and R⁴ as well as the variable n are the same as defined above. This product (compound 3) is further reacted with a compound of formula H₂N-R¹-SH (compound 4). Group R¹ is an alkylene or a heteroalkylene with one or more -O-, -S-, or -NH- heteroatoms. In many embodiments, the group R¹ is an alkylene having 2 to 10 carbon atoms. The number of carbon atoms can be at least 2, at least 4, or at least 6 and up to 10, up to 8, or up to 6.

In many embodiments, the compound H₂N-R¹-SH (compound 4) is 2-aminoethane thiol with R¹ being ethylene. The final reaction product of Reaction Scheme A is the compound R²-(NH-CO-CO-NH-R¹-SH)ₙ where R¹ is an alkylene. The group R² as well as the variable n are the same as described above. This final product is a compound of Formula (I-1).

The compounds of Formula (I) can be formed in a similar manner as described in Reaction Scheme A, but an additional diamine compound is added during the synthesis. The additional diamine is of formula NH₂-R³-NH₂. This additional diamine is typically added using any one of several possible approaches. For example, in a first approach, the additional diamine can be added as shown in Reaction Scheme B. Some of the reaction byproducts are not shown in this reaction scheme.

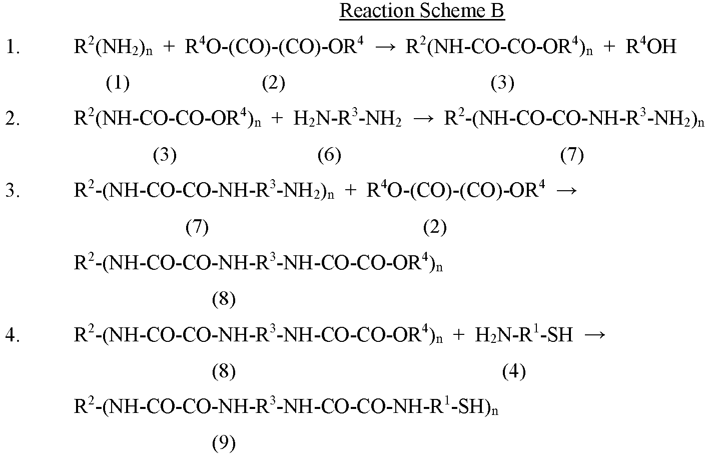

In Reaction Scheme B, the first step is like that of Reaction Scheme A where the polyamine (compound 1) of formula R²(NH₂)ₙ is reacted with an oxalate compound (compound 2) of formula R⁴O-(CO)-(CO)-OR⁴ to form a compound of formula R²(NH-CO-CO-OR⁴)ₙ (compound 3). In the second step, compound (3) is reacted with a diamine of formula H₂N-R³-NH₂ (compound 6). Any diamine such as those described above for use in Reaction Scheme A can be used. In the third step, the product of the second reaction step, which is R²-(NH-CO-CO-NH-R³-NH₂)ₙ (compound 7), is reacted the oxalate compound (compound 2) to produce compound 8 of formula R²-(NH-CO-CO-NH-R³-NH-CO-CO-OR⁴)ₙ. Compound 8 is then reacted with a compound of formula H₂N-R¹-SH (compound 4) as described for Reaction Scheme A to form compound 9 of formula R²-(NH-CO-CO-NH-R³-NH-CO-CO-NH-R¹-SH)ₙ. The alcohol R⁴OH is formed as a byproduct but is not shown in the second, third, and fourth reaction.

Another method of forming the compound of Formula (I) is shown in Reaction Scheme C. 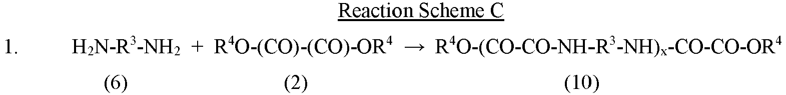 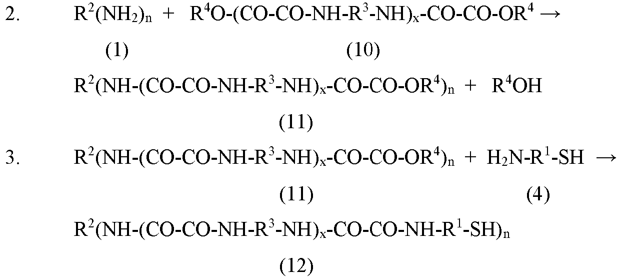 In this reaction scheme, the compound of formula H₂N-R³-NH₂ (compound 6) can initially be reacted with the oxalate compound of formula R⁴O-(CO)-(CO)-OR⁴ (compound 2) to form a compound of formula R⁴O-(CO-CO-NH-R³-NH)ₓ-CO-CO-OR⁴ (compound 10). The groups R⁴ and R³ plus the variable x are the same as defined above. Next, compound 10 of formula R⁴O-(CO-CO-NH-R³-NH)ₓ-CO-CO-OR⁴ is reacted with the polyamine of formula R²(NH₂)ₙ (compound 1) to form the compound of formula R²(NH-(CO-CO-NH-R³-NH)ₓ-CO-CO-OR⁴)ₙ (compound 11). Compound 11 is then reacted with H₂N-R¹-SH (compound 4) to form a compound of formula R²(NH-(CO-CO-NH-R³-NH)ₓ-CO-CO-NH-R¹-SH)ₙ (compound 12). Compound 12 is a compound of Formula (I) where R¹ is an alkylene.

In addition to the Reaction Schemes A to C, any other known method may be used to form the compounds of Formula (I) and/or Formula (I-1).

### Reaction mixtures and polymerized product thereof

The thiol-containing compounds containing at least two groups of formula -NH-CO-CO-NH-R¹-SH can be reacted with an epoxy resin to form a polymerized product. The polymerized product can be either a thermoplastic or thermoset material.

The thiol-containing compound tends to react quickly with the epoxy resin. Thus, the reaction mixture can be either a single composition that includes both the thiol-containing compound and the epoxy resin or can be divided into a fist part and a second part. Single reaction mixtures that include both the thiol-containing compound and an epoxy resin are typically intended to be used immediately after preparation. In many embodiments, the reaction mixture includes a first part comprising the thiol-containing compound described above, which is often of Formula (I), and a second part that comprises the epoxy resin. The first and second parts can be prepared prior to the intended time of use and can be stored for extended periods prior to being combined.

The first part that contains the thiol-containing compound can optionally further include a curing catalyst. Examples of curing catalysts include, but are not limited to, phenols substituted with one or more tertiary amino groups, amidine bases, bis-substituted urea compounds, imidazole compounds, imidazoline compounds, sulfonic acid compounds, Lewis acids, and salts of any of these compounds.

Any suitable epoxy resin can be used in the reaction mixture and/or in the second part of a two-part reaction mixture, but it typically has at least two glycidyl groups. In most embodiments, the epoxy resins are liquids at temperatures less than about 40 degrees Celsius such as at room temperature (e.g., 20 to 25 degrees Celsius). A mixture of different epoxy resins can be used, including solid epoxy resins, if the mixture and/or the second part is a liquid at temperatures less than 40 degrees Celsius such as at room temperature.

Suitable epoxy resins may include aromatic polyepoxide resins (e.g., a chain-extended diepoxide or novolac epoxy resin having at least two epoxide groups), aromatic monomeric diepoxides, aliphatic polyepoxides, or aliphatic monomeric diepoxides. The aromatic polyepoxide or aromatic monomeric diepoxide typically contains at least one (e.g., in a range of 1 to 6, 1 to 4, 2 to 6, or 2 to 4) aromatic ring that is optionally substituted by a halogen (e.g., fluoro, chloro, bromo, iodo), alkyl having 1 to 4 carbon atoms (e.g., methyl or ethyl), or hydroxyalkyl having 1 to 4 carbon atoms (e.g., hydroxymethyl). For epoxy resins containing two or more aromatic rings, the rings may be connected, for example, by a branched or straight-chain alkylene group having 1 to 4 carbon atoms that may optionally be substituted by halogen (e.g., fluoro, chloro, bromo, iodo).

Examples of aromatic epoxy resins include novolac epoxy resins (e.g., phenol novolacs, ortho-, meta-, or epoxy resin para-cresol novolacs, or combinations thereof), bisphenol epoxy resins (e.g., bisphenol A, bisphenol F, halogenated bisphenol epoxies, or combinations thereof), resorcinol epoxy resins, tetrakis phenylolethane epoxy resins, or combinations of any of these. Useful epoxy compounds include diglycidyl ethers of difunctional phenolic compounds (e.g., p,p'-dihydroxydibenzyl, p,p'-dihydroxydiphenyl, p,p'-dihydroxyphenyl sulfone, p,p'-dihydroxybenzophenone, 2,2'-dihydroxy-1,1-dinaphthylmethane, and the 2,2', 2,3', 2,4', 3,3', 3,4', and 4,4' isomers of dihydroxydiphenylmethane, dihydroxydiphenyldimethylmethane, dihydroxydiphenylethylmethylmethane, dihydroxydiphenylmethylpropylmethane, dihydroxydiphenylethylphenylmethane, dihydroxydiphenylpropylphenylmethane, dihydroxydiphenylbutylphenylmethane, dihydroxydiphenyltolylethane, dihydroxydiphenyltolylmethylmethane, dihydroxydiphenyldicyclohexylmethane, and dihydroxydiphenylcyclohexane). In some embodiments, the epoxy resin includes a bisphenol diglycidyl ether, wherein the bisphenol (i.e., -O-C₆H₃-CH₂-C₆H₃-O- group) may be unsubstituted (e.g., bisphenol F), or wherein either of the phenyl rings or the methylene group may be substituted by one or more halogens (e.g., fluoro, chloro, bromo, iodo), methyl groups, trifluoromethyl groups, or hydroxymethyl groups.

Examples of aromatic monomeric diepoxides useful as the epoxy resin include, but are not limited to, the diglycidyl ether of bisphenol A, the diglycidyl ether of bisphenol F, and mixtures thereof. Bisphenol epoxy resins, for example, may be chain extended to have any desirable epoxy equivalent weight. Chain extending epoxy resins can be carried out by reacting a monomeric diepoxide, for example, with a bisphenol in the presence of a catalyst to make a linear polymer.

The aromatic epoxy resin (e.g., either a bisphenol epoxy resin or a novolac epoxy resin) often has an epoxy equivalent weight of at least 150 grams per equivalent, at least 170 grams per equivalent, at least 200 grams per equivalent, or at least 225 grams per equivalent. The epoxy equivalent weight can be up to 2000 grams per equivalent, up to 1500 grams per equivalent, or up to 1000 grams per equivalent. In some embodiments, the aromatic epoxy resin may have an epoxy equivalent weight in a range of 150 to 2000 grams per equivalent, 150 to 1000 grams per equivalent, or 170 to 900 grams per equivalent. For example, the epoxy resin can have an epoxy equivalent weight in a range of 150 to 450 grams per equivalent, 150 to 350 grams per equivalent, or 150 to 300 grams per equivalent. Epoxy equivalent weights may be selected, for example, so that the epoxy resin may be used as a liquid or solid, as desired.

In some embodiments, in addition or as an alternative to aromatic epoxy resins, the epoxy resins may include one or more non-aromatic epoxy resins. In some cases, non-aromatic (i.e., aliphatic) epoxy resins can be useful as reactive diluents that may help control the flow characteristics of the compositions. Non-aromatic epoxy resins useful in the curable compositions include, for example, a branched or straight-chain alkylene group having 1 to 20 carbon atoms optionally interrupted with at least one -O- and optionally substituted by hydroxyl. In some embodiments, the non-aromatic epoxy can include a poly(oxyalkylene) group having a plurality (q) of oxyalkylene groups, -OR⁶-, wherein each R⁶ is independently an alkylene having 2 to 5 carbon atoms. In some embodiments, R⁶ is an alkylene with 2 to 4 carbon atoms and q is 2 to about 6 (or even higher) such as 2 to 5, 2 to 4, or 2 to 3. To become crosslinked into a network, useful non-aromatic epoxy resins will typically have at least two epoxy end groups.

Examples of useful non-aromatic epoxy resins include glycidyl epoxy resins such as those based on diglycidyl ether compounds comprising one or more oxyalkylene units. Examples of these epoxy resins include ethylene glycol diglycidyl ether, propylene glycol diglycidyl ether, diethylene glycol diglycidyl ether, dipropylene glycol diglycidyl ether, polyethylene glycol diglycidyl ether, polypropylene glycol diglycidyl ether, glycerol diglycidyl ether, glycerol triglycidyl ether, propanediol diglycidyl ether, butanediol diglycidyl ether, and hexanediol diglycidyl ether. Other useful non-aromatic epoxy resins include a diglycidyl ether of cyclohexane dimethanol, a diglycidyl ether of neopentyl glycol, a triglycidyl ether of trimethylolpropane, and a diglycidyl ether of 1,4-butanediol.

Several suitable epoxy resins are commercially available. For example, several epoxy resins of various classes and epoxy equivalent weights are available from Dow Chemical Company (Midland, MI, USA), Hexion, Inc. (Columbus, OH, USA), Huntsman Advanced Materials (The Woodlands, TX, USA), CVC Specialty Chemicals Inc. (Akron, OH, USA and recently acquired by Emerald Performance Materials), and Nan Ya Plastics Corporation (Taipei City, Taiwan). Examples of commercially available glycidyl ethers include diglycidyl ethers of bisphenol A (e.g., those available under the trade designations "EPON" from Hexion Inc. (Columbus, OH, USA) (e.g., EPON 828, EPON 1001, EPON 1310, and EPON 1510), those available under the trade designation "D.E.R." from Dow Chemical Co. (e.g., D.E.R. 331, 332, and 334), those available under the trade designation "EPICLON" from Dainippon Ink and Chemicals, Inc. (e.g., EPICLON 840 and 850), and those available under the trade designation "YL-980" from Japan Epoxy Resins Co., Ltd.)); diglycidyl ethers of bisphenol F (e.g., those available under the trade designation "EPICLON" from Dainippon Ink and Chemicals, Inc. (e.g., EPICLON 830)); polyglycidyl ethers of novolac resins (e.g., novolac epoxy resins, such as those available under the trade designation "D.E.N." from Dow Chemical Co. (e.g., D.E.N. 425, 431, and 438)); and flame retardant epoxy resins (e.g., D.E.R. 580, a brominated bisphenol type epoxy resin available from Dow Chemical Co.). Examples of commercially available non-aromatic epoxy resins include the diglycidyl ether of cyclohexane dimethanol, available from Hexion Inc. (Columbus OH, USA) under the trade designation HELOXY MODIFIER 107.

The reaction mixture typically contains a ratio of the moles of active hydrogen (from -SH groups) to moles of epoxy groups that is in a range of 2:1 to 1:2. For example, the ratio can be in a range of 1.5:1 to 1:1.5, in a range of 1.3:1 to 1:1.3 or in a range of 1.2:1 to 1:1.2. In many embodiments, however, the ratio is selected so that there is an excess of active hydrogen groups. That is, the range is from 2:1 to 1.1:1, from 2:1 to 1.2:1, from 2:1 to 1.3:1, or from 2:1 to 1.5:1.

The polymerized product of the reaction mixture can be either a thermoplastic or thermoset material. If a thermoplastic material is desired, the thiol-containing compound typically is selected to have two thiol-containing groups of formula -NH-(CO)-(CO)-NH-R¹-SH and the epoxy resin typically is selected to have two oxirane groups. These reaction mixtures typically are free of thiol-containing compounds with more than two thiol groups and epoxy resins with more than two oxirane groups but can include additional dithiol compounds that do not have groups of formula -NH-(CO)-(CO)-NH-R¹-SH.

Optional dithiol compounds without -NH-(CO)-(CO)-NH-R¹-SH groups, however, can be included in the reaction mixtures used to form thermoplastic materials. These additional optional dithiol compounds can be added, for example, to modify the thermal properties of the thermoplastic material. For example, the glass transition temperature of the thermoplastic can decrease with the addition of a dithiol lacking groups of formula -NH-(CO)-(CO)-NH-R¹-SH. That is, such a dithiol compound can be added to adjust the glass transition temperature to one that is favorable for a particular application. The dithiol compounds can also be added to adjust the viscosity of the reaction mixture prior to polymerization. Suitable dithiols include, for example, those of formula HS-R⁵-HS where R⁵ comprises an alkylene or heteroalkylene and can optionally further comprise a carbonyl group and/or a carbonyloxy (-(CO)-O-) group. The alkylene and heteroalkylene groups can be linear, branched, cyclic, or a combination thereof. The heteroalkylene can have heteroatoms selected from -O-, -NH-, -S-, or combinations thereof.

Example dithiol compounds HS-R⁵-HS include, for example, 1,2-ethanedithiol, 1,2-propanedithiol, 1,3-propanedithiol, 1,3-butanedithiol, 1,4-butanedithiol, 2,3-butanedithiol, 1,3-pentanedithiol, 1,5-pentanedithiol, 1,6-hexanedithiol, 1,3-dimercapto-3-methylbutane, dipentenedimercaptan, ethylcyclohexyldithiol (ECHDT), 1,5-dimercapto-3-oxapentane, 1,8-dimercapto-3,6-dioxaoctane, 2,2'-(ethylenedioxy)diethanetliiol, tetra(ethylene glycol) dithiol, and hexa(ethylene glycol) dithiol. Suitable thiols with carbonyloxy groups are various ester-containing compounds such as ethylene glycol bis-mercaptoacetate and ethylene glycol bis-mercaptopropionate.

If a thermoset material is desired, then the reaction mixture usually contains at least one of the following: a thiol-containing compound having at least three thiol-containing groups that are of formula -NH-(CO)-(CO)-NH-R¹-SH, a thiol-containing compound having at least three thiol groups that are not of formula -NH-(CO)-(CO)-NH-R¹-SH, or an epoxy resin having at least three oxirane groups.

In some embodiments, the thermoset material is prepared using a thiol-containing compound having at last three thiol groups that are not of formula -NH-(CO)-(CO)-NH-R¹-SH. This compound can be used to adjust the viscosity of the curable composition and adjust the thermal properties of the cured composition. Suitable thiol-containing compounds having at least three thiol groups that are not of formula -NH-(CO)-(CO)-NH-R¹-SH include, for example, pentaerythritol tetrakis(3-mercaptopropionate), trimethylolpropane tris(3-mercaptopropionate), tris[2-(3-mercaptopropionyloxy)ethyl]isocyanurate (TEMPIC), dipentaerythritol hexakis(3-mercaptopropionate), and the like.

Other optional components can be added to the first part, to the second part, or to both parts provided they do not react with other components in that part.

In some curable compositions, an optional organic solvent is included in the first part, in the second part, or in both parts. Suitable organic solvents include, but are not limited to, methanol, tetrahydrofuran, ethanol, isopropanol, pentane, hexane, heptane, acetone, methyl ethyl ketone, methyl acetate, ethyl acetate, toluene, xylene, ethylene glycol alkyl ether, propylene carbonate, and mixtures thereof. The organic solvent can be added to dissolve a component in the curable composition or can be added to lower the viscosity of the curable composition to facilitate its dispensing. The amount of the organic solvent in the curable composition can be in a range of 0 to 10 weight percent based on a total weight of the curable composition. In some embodiments, the amount is at least 0.5 weight percent, at least 1 weight percent, at least 2 weight percent, at least 3 weight percent, at least 4 weight percent and up to 10 weight percent, up to 9 weight percent, up to 8 weight percent, up to 7 weight percent, up to 6 weight percent, or up to 5 weight percent. In many embodiments, no optional organic solvent is included in the first part, in the second part, or in either part.

The curable composition optionally contains a flow control agent or thickener, to provide the desired rheological characteristics to the composition. Silica is a thixotropic agent and is added to provide shear thinning. Silica has the effect of lowering the viscosity of the curable composition when force (shear) is applied. When no force (shear) is applied, however, the viscosity seems higher. That is, the shear viscosity is lower than the resting viscosity. The silica typically has a longest average dimension that is less than 500 nanometers, less than 400 nanometers, less than 300 nanometers, less than 200 nanometers, or less than 100 nanometers. The silica particles often have a longest average dimension that is at least 5 nanometers, at least 10 nanometers, at least 20 nanometers, or at least 50 nanometers. In some embodiments, the silica particles are fumed silica such as treated fumed silica, available under the trade designation CAB-O-SIL TS 720, and untreated fumed silica available under the trade designation CAB-O-SIL M5, from Cabot Corporation (Alpharetta, GA, USA). In other embodiments, the silica particles are non-aggregated nanoparticles.

If used, the amount of the optional silica particles is at least 0.5 weight percent based on a total weight of the curable composition. The amount of the silica can be at least 1 weight percent, at least 1.5 weight percent, or at least 2 weight percent and can be up to 10 weight percent, up to 8 weight percent, or up to 5 weight percent. For example, the amount of silica can be in a range of 0 to 10 weight percent, 0.5 to 10 weight percent, 1 to 10 weight percent, 0.5 to 8 weight percent, 1 to 8 weight percent, 0.5 to 5 weight percent, or 1 to 5 weight percent.

The curable composition can optionally include fibers for reinforcement of the cured composition. However, in many embodiments, the curable compositions are free or substantially free of fiber reinforcement. As used herein, "substantially free" means that the curable compositions contain no greater than 1 weight percent, no greater than 0.5 weight percent, no greater than 0.2 weight percent, no greater than 0.1 weight percent, no greater than 0.05 weight percent, or no greater than 0.01 weight percent of fibers.

In some embodiments, the curable composition optionally contains adhesion promoters to enhance the bond to the substrate. The specific type of adhesion promoter may vary depending upon the composition of the surface to which it will be adhered. Various silane and titanate compounds have been used to promote adhesion to the first substrate and/or the second substrate that are bonded together with the cured composition. If present, the amount of the adhesive promoter would be up to 5 weight percent, up to 3 weight percent, up to 2 weight percent, or up to 1 weight percent and at least 0.1 weight percent, at least 0.2 weight percent, or at least 0.5 weight percent based on the total weight of the curable composition.

Still other optional components include, for example, fillers (e.g., aluminum powder, carbon black, glass bubbles, talc, clay (e.g., montmorillonite, bentonite, or halloysite), calcium carbonate, calcium triflate, barium sulfate, titanium dioxide, and mica), stabilizers, plasticizers, tackifiers, cure rate retarders, impact modifiers, toughening agents (e.g., core-shell rubbers), expandable microspheres, glass beads or bubbles, thermally conductive particles, electrically conductive particles, corrosion inhibitors, fire retardants, antistatic materials, glass, pigments, colorants, waxes (e.g., hydrogenated oils or amides), UV stabilizers, and antioxidants. The optional components can be added, for example, to reduce the weight of the structural adhesive layer, to adjust the viscosity, to provide additional reinforcement, to modify the thermal or conductive properties, to alter the rate of curing, and the like. If any of these optional components are present, they are typically used in an amount that does not prevent the printing or dispensing of the curable composition.

Any of these additional optional components can be in the first part, the second part, or both providing they do not result in substantial curing of other components in these parts.

The curable composition, which includes both the first part and the second part, is mixed, and reacted to form the cured composition. That is, the cured composition is a reaction product of the first and second parts. The first and second parts are typically fluids at the mixing temperature, which is often no greater than 40 degrees Celsius such as at or near room temperature (e.g., 20 to 25 degrees Celsius). Optional additional parts can be mixed with the first part and the second part. Any suitable method can be used to combine the various parts of the curable compositions.

The first and second parts can be mixed manually or with any known mechanical mixing and/or dispensing device. For example, the first part can be in a first chamber and the second part can be in a second chamber of a multi-chambered mixing and/or dispensing device. In certain embodiments, the multi-chambered mixing and/or dispensing device is a dual barreled syringe. Optionally, the dual barreled syringe may include or be connected to a static mixing device to mix the contents of each barrel upon delivery from the syringe and prior to discharging the cured composition (i.e., mixed composition) on the location of interest. While some curing may occur within the mixing device, the reaction mixture is typically still fluid when discharged from the mixing device. Although not required, the viscosity of the first part and the second part are often selected to be similar so that the part can be effectively mixed.

The mixed composition can be discharged on any suitable surface and the resulting cured composition is often used to adhere a first substrate to a second substrate. The cured composition is usually a structural adhesive. The various substrates that are joined can be the same or different and often are selected from polymeric material, glass or ceramic material, metal or metal oxide materials, and the like.

### Examples

Unless otherwise noted, all parts, percentages, ratios, etc. in the Examples and the rest of the specification are by weight. Unless otherwise indicated, all other reagents were obtained, or are available from fine chemical vendors such as Sigma-Aldrich Company, St. Louis, Missouri, or may be synthesized by known methods. Table 1 (below) lists materials used in the examples and their sources.

**TABLE 1. Materials List**

| Material | Description | Source |
|---|---|---|
| DEO | Diethyl Oxalate; CAS# 95-92-1 | Alfa Aesar, Tewksbury, MA |
| D400 | Difunctional amine-terminated polyether obtained under the trade designation JEFFAMINE D-400 Polyetheramine | Huntsman Corporation, The Woodlands, TX, USA |
| D230 | Difunctional amine-terminated polyether obtained under the trade designation JEFFAMINE D-230 Polyetheramine | Huntsman Corporation, The Woodlands, TX, USA |
| T403 | Trifunctional amine-terminated polyether obtained under the trade designation JEFFAMINE D-230 Polyetheramine | Huntsman Corporation, The Woodlands, TX, USA |
| TTD | 4,7,10-Trioxa-1,13-tridecancediamine | TCI America, Portland, OR, USA |
| IPDA | Isophorone diamine *cis, trans* mixture; CAS# 2855-13-2 | TCI America, Portland, OR, USA |
| PACM | 4,4'-Methylenebis(cyclohexylamine) as a mixture of isomers obtained under the trade designation VESTAMIN PACM from Evonik | Evonik Industries AG, Essen, Germany |
| TMD | 2,2,4-timethyl-1,6-diaminoxexane and 2,4,4-trimethyl-1,6-diaminohexane. Mixture of isomers obtained under the trade designation VESTAMIN TMD from Evonik | Evonik Industries AG, Essen, Germany |
| AET | 2-aminoethane thiol; CAS# 60-23-1 | TCI America, Portland, OR, USA |
| DMDO | 2,2'-(Ethylenedioxy)diethanethiol; CAS# 14970-87-7 | TCI America, Portland, OR, USA |
| TMPMP | Trimethylolpropane tris(3-mercaptopropionate); CAS# 33007-83-9 | TCI America, Portland, OR, USA |
| EPON 828 | BPA Epoxy solution obtained under the trade designation EPON 828 | Hexion Inc. |
| AK54 | 2,4,6-tris-(dimethylaminomethyl)phenol | TCI America, Portland, Oregon |
| Allylamine | 3-Amino-1-propene; CAS# 107-11-9 | TCI America, Portland, Oregon |
| DMPA | 2,2-dimethoxy-2-phenylacetophenone; CAS# 24650-42-8 | Acros Organics; New Jersey, USA |
| Chloroform | CAS# 67-66-3 | MilliporeSigma, Burlington MA, USA |
| Ethanol | CAS# 64-17-5 | MilliporeSigma, Burlington MA, USA |
| DMF | N,N-Dimethylformamide; CAS # 68-12-2 | MilliporeSigma, Burlington MA, USA |
| TS720 | Hydrophobic fumed amorphous silica. Obtained under the trade designation CAB-O-SIL TS-720 from Cabot Corporation | Cabot Corporation; Alpharetta GA, USA |
| DBU | 1,8-Diazabicyclo[5.4.0]undec-7-ene | Alfa Aesar, Tewksbury, MA |

### Test Methods

### Rheometric Test Method

The viscoelastic characteristics of the were measured using a TA Instruments (New Castle, DE) Discovery HR-3 rheometer. A circular die punch 8 mm in diameter was used to cut individual test specimens from the pressed films. Measurements were performed with parallel plate geometry utilizing 8 mm diameter steel tools and an Environmental Test Chamber system (TA Instruments, New Castle, DE) for temperature control. Oscillatory temperature ramp experiments were performed from 150 to -40 °C at a ramp rate of 2.0 °C/min. using 0.5 % strain and 1 Hz frequency. A dwell time of 180 seconds at 150 °C was used to ensure thermal equilibrium in each sample before each temperature ramp and a constant compressive force of 0.2 ± 0.4 N thorough the experiments to maintain contact between the specimen and instrument during data collection. The glass transition temperature (Tg) is reported as the local maxima in the loss tangent (Tan(δ)) observed during the transition from rubbery to glassy solid.

### Dynamic Mechanical Analysis Test Method

A TA Instruments RSA-G2 solids analyzer (obtained from TA Instruments, New Castle, DE) equipped with a tension film fixture was used to measure the viscoelastic characteristics of the prepared films. Rectangular test samples were cut from prepared films with approximate dimensions of 40 mm x 6.20 mm x 1.00 mm. The in-phase and out-of-phase deformation response was measured while applying a sinusoidal strain of 0.1 % at a frequency of 1 Hz. The resulting storage and loss moduli and loss tangent were calculated. The temperature was ramped at 2 °C/min over a temperature range from -40 to 150 °C. The glass transition temperature (Tg) is reported as the local maxima in the loss tangent (Tan(δ)) observed during the transition from rubbery to glassy solid.

### Elongation Test Method

Curable mixtures were coated between polyester release liners at approximately 1mm thickness. The coated films cured for at least 18 hours at 23 °C, followed by 90°C for 1 h and cooled to room temperature before testing. Test samples were cut from the cured films using a TYPE-V die as specified in ASTM Standard D638 - 14 "Standard Test Method for Tensile Properties of Plastics". The samples were tested to failure in uniaxial tension at a rate of 50 mm/min using a tensile load frame with pneumatically tightened grips (MTS Systems, Eden Prairie, MN). The tensile modulus value was taken as the initial liner slope of the stress vs strain curve and elongation was taken as the strain at break expressed as a percentage of the initial gauge length of the sample and the displacement of the crosshead during testing. The average of five individual test samples is reported.

### Overlap Shear Test Method

Aluminum coupons (1"x4"x0.062") were wiped with isopropyl alcohol abraded with wet or dry sandpaper and rinsed with isopropyl alcohol a second time . The adhesive mixture was then applied to a 1"x0.5" area on one end of the aluminum coupon, and 0.005 inch (0.127 mm) glass spacer beads were incorporated into the test formulations as noted in the table below to control bond line thickness. One end of a second aluminum coupon was then pressed into to the mixture to produce an overlap of approximately 0.5". A binder clip was placed on the sample, and it was cured for at least 18 hours at 23 °C, followed by 90°C for 1 h and cooled to room temperature before testing. The samples were tested to failure in uniaxial tension at a rate of 0.1 in/min using a tensile load frame with Advantage Wedge Action Grips (MTS Systems, Eden Prairie, MN). The single lap shear strength was taken as the peak force recorded during testing divided by the overlap area of the two coupons. The average of five individually prepared samples is reported.

Polycarbonate coupons (1"x 4"x 0.125") were used. Adhesive mixtures were then applied to a 1 inch by 0.5 inch area on one end of a coupon, and 0.005 inch (0.127 mm) glass spacer beads were incorporated into the test formulations as noted in the table below to control bond line thickness. One end of a second coupon was then pressed into to the mixture to produce an overlap of approximately 0.5 inches. A binder clip was placed on the sample, and it was cured for at least 72 hours at 23 °C. The samples were tested to failure in uniaxial tension at a rate of 2.0 in/min using a tensile load frame with Advantage Wedge Action Grips (MTS Systems, Eden Prairie, MN). The single lap shear strength was taken as the peak force recorded during testing divided by the overlap area of the two coupons. The average of five individually prepared samples is reported.

### Nuclear Magnetic Resonance (NMR)

A portion of the oxamide-thiol materials and other polythiols were analyzed as a solution of unknown concentration (generally approximately 12 milligrams/milliliter (mg/mL)) in dimethyl sulfoxide-D6. NMR spectra were acquired on a Bruker AVANCE 600 megahertz (MHz) NMR spectrometer equipped with an inverse cryoprobe.

¹H-NMR analysis was used to confirm molecular structure, determine the molecular weight, and amine (-SH) equivalents for each of the oxamide-thiol and polythiol materials used.

### Preparation of thiol-oxamide compounds: Preparatory Examples PE-1 to PE-7

The thiol-terminated oxamide compounds, listed in Table 2, were prepared according to the following general procedure using the amounts of reagents listed in Table 2.

In a first step, diethyl oxalate (DEO) and half of the total volume of chloroform were added to a round-bottomed flask with a magnetic stir bar. The flask was fitted with a pressure equalizing addition funnel loaded with the specified amine and the remaining volume of chloroform. The total volume of chloroform was selected so that the total reaction mixture would have an amine concentration of 1 mole per liter. The apparatus was placed under an inert atmosphere of nitrogen gas and stirred at 400 rpm. The amine solution was added dropwise, approximately 1 drop per second, to the DEO solution. After addition was complete, the addition funnel was rinsed with 10 mL of chloroform. The reaction mixture was stirred at 23-25 °C for 18-20 hours before the solvent, excess DEO, and the ethanol evolved during reaction was removed under reduced pressure at 80 °C using a ROTAVAPOR R-100 rotary evaporator (BUCHI Corporation, New Castle, DE, USA).

In a second step, the product of the first step was then dissolved in 100 mL of chloroform and AET was added as specified in Table 2. This reaction flask was fitted with a water-cooled condenser and was heated to reflux using a silicone oil bath set to 90 °C. The reaction was refluxed for two hours and then removed from the oil bath and cooled. The solid precipitated products were collected via vacuum filtration except for D400-AET and T403-AET, which are high viscosity liquids. These two materials were isolated by removing all solvent, excess AET, and ethanol under reduced pressure at 80 °C using a ROTAVAPOR R-100 rotary evaporator (BUCHI Corporation, New Castle, DE, USA).

**TABLE 2. Thiol-oxamide formulations for Preparative Examples PE-1 to PE-7.**

| Preparative Example | Name | Amine | Amine Mass, g | Mass DEO, g | Mass AET, g |
|---|---|---|---|---|---|
| PE-1 | TTD-AET | TTD | 55.01 | 109.62 | 48.22 |
| PE-2 | IPDA-AET | IPDA | 42.59 | 109.06 | 44.65 |
| PE-3 | PACM-AET | PAMC | 52.64 | 111.23 | 46.33 |
| PE-4 | TMD-AET | TMD | 31.68 | 64.31 | 25.32 |
| PE-5 | D400-AET | D400 | 125.05 | 85.02 | 46.16 |
| PE-6 | T403-AET | T403 | 28.20 | 31.71 | 14.21 |
| PE-7 | D230-AET | D230 | 48.09 | 117.40 | 46.60 |

### Preparation of thermoplastic polymers from compositions of epoxy and thiol-oxamide compounds: Comparative Example CE-1 and Examples EX-1 to EX-2

Thermoplastic polymers were prepared by combining the materials listed in Table 3 except DBU in a 70 mm diameter aluminum pan and heating on a hot plate set to 170 °C. Once the mixture was homogeneous, DBU was added in the amount indicated in Table 3 and the curable compositions were mixed by hand with a wooden spatula. After 30 minutes at temperature, the samples were cooled to ambient temperature and removed from the aluminum pans. The thermoplastic polymers thus created were pressed to a thickness of 1.00 ± 0.10 mm between two sheets of polyester release liner using steel shims and a hydraulic press (Model 2699 Carver, Inc., Wabash, IN, USA) heated to 121 °C. The films thus created were used to determine the glass transition temperature (T_{g}) via the dynamic mechanical analysis test method described above.

**TABLE 3. Thermoplastic polymer compositions and glass transition temperatures**

| | EPON 828 (g) | DMDO (g) | PE-1 (g) | DBU (g) | T_{g} (°C) |
|---|---|---|---|---|---|
| CE-1 | 5.67 | 2.73 | | 0.08 | 18 |
| EX-1 | 5.67 | 1.37 | 3.62 | 0.11 | 39 |
| EX-2 | 5.67 | | 7.24 | 0.13 | 44 |

The glass transition values increased with the incorporation of oxamide units in the backbone of the linear polymers synthesized.

### Preparation of thermoset polymers from compositions of epoxy and thiol-oxamide compounds: Comparative Example CE-2 and Examples EX-3 to EX-9

Thermosetting formulations were prepared in a MAX 40 polypropylene cup (FlackTeck Inc., Landrum SC, USA) according to the masses listed in Table 4. All components except EPON 828 and K54 were placed into a cup and heated in an oven under nitrogen gas to 120 °C until a homogeneous solution was obtained. EPON 828 was then added, and the samples were mixed in a Dual Asymmetric Centrifuge (DAC) SPEEDMIXER (FlackTek Inc., Landrum, South Carolina) for 2 minutes at 2500 rpm. Samples were cooled to ambient temperature (approximately 23 °C) before K54 (a catalyst) was added and the formulation was mixed again for 30 seconds at 2500 rpm.

Films of examples were prepared by knife coating the mixtures between two sheets of polyester release liner using a 6-inch-wide knife set at a gap of 1.00 mm. The coated films cured for at least 18 hours at 23 °C, followed by 90 °C for 1 hour and cooling to room temperature before testing according to the methods above for thermoset T_{g} and tensile properties.

**TABLE 4. Summary of the formulations for CE-2 and EX-4 to EX-8**

| | CE-2 | EX-4 | EX-5 | EX-6 | EX-7 | EX-8 | EX-9 |
|---|---|---|---|---|---|---|---|
| | Mass, g | Mass, g | Mass, g | Mass, g | Mass, g | Mass, g | Mass, g |
| EPON 828 | 9.45 | 9.45 | 9.45 | 9.45 | 9.45 | 9.45 | 9.45 |
| TS720 | 0.88 | 0.90 | 0.88 | 0.98 | 0.9 | 0.88 | |
| PE-1 | | 3.45 | | | | | |
| PE-2 | | | 3.09 | | | | |
| PE-3 | | | | | 3.38 | | |
| PE-4 | | | | | | 3.00 | |
| PE-5 | | | | 4.95 | | | |
| PE-7 | | | | | | | 10.75 |
| TMPMP | 6.64 | 0.95 | 0.95 | 0.95 | 0.95 | 0.95 | 0.95 |
| DMDO | | 2.60 | 2.60 | 2.60 | 2.60 | 2.60 | |
| K54 | 0.66 | 0.66 | 0.66 | 0.66 | 0.66 | 0.66 | 0.66 |
| Total Mass (g) | 17.64 | 18.01 | 17.64 | 19.59 | 17.04 | 16.67 | 21.81 |

**TABLE 5. Tensile and dynamic mechanical analysis data: CE-2 and EX-4 to EX-9**

| | Tensile Modulus, MPa (PSI) | Tensile Elongation, % | T_{g}, °C |
|---|---|---|---|
| CE-2 | 5317 (771200) | 1.09 | 34 |
| EX-4 | 785.5 (114000) | 4.50 | 40 |
| EX-5 | 5992 (869000) | 0.62 | 67 |
| EX-6 | 10.38 (1506) | 165.81 | 31 |
| EX-7 | 5648 (819200) | 0.58 | 58 |
| EX-8 | 5861 (850100) | 0.76 | 55 |
| EX-9 | 2293 (332700) | 1.83 | 61 |

Adding the oxamide-containing dithiol tends to increase the glass transition temperature without a substantial decrease in the elongation or tends to increase the elongation without a substantial decrease in the glass transition temperature.

### Adhesive Strength by Single Lap Shear: Comparative Example CE-3 and Examples EX-10 to EX-13

Curable compositions were prepared according to the masses listed in Table 6 below and were evaluated according to the elongation and overlap shear test methods described above. Commercially available epoxy-thiol two-part adhesive 3M SCOTCH-WELD Epoxy Adhesive DP100 was used as comparative example (CE-3). This commercial material uses a non-oxamide containing multifunctional thiol as a curative for an epoxy base resin. The test results for each of the examples EX-10 to EX-13 and CE-3 are tabulated in Table 7 below.

**TABLE 6. Curable compositions of examples 10-13.**

| | EX-10 | EX-11 | EX-12 | Ex-13 |
|---|---|---|---|---|
| | Mass (g) | Mass (g) | Mass (g) | Mass (g) |
| EPON 828 | 18.95 | 18.93 | 18.94 | 18.91 |
| TS720 | 0.60 | 0.60 | 0.60 | 0.60 |
| 5 mil Beads | 0.50 | 0.50 | 0.50 | 0.50 |
| PE-2 | 0.88 | 1.67 | 3.06 | 6.13 |
| TMPMP | 1.99 | 1.99 | 1.99 | 1.99 |
| DMDO | 7.38 | 7.04 | 6.46 | 5.17 |
| K54 | 0.66 | 0.66 | 0.66 | 0.66 |
| Total Mass (g) | 30.96 | 31.40 | 32.22 | 33.96 |

**TABLE 7: Overlap shear and tensile data for comparative example 3 and examples 10-13.**

| | T_{g} (°C) | Substrate | Single lap shear strength, MPa (PSI) | Tensile Modulus, MPa (PSI) | Tensile Elongation, % |
|---|---|---|---|---|---|
| CE-3 | 54.35 | Aluminum | 4.159 (603.2) | 2538 (368000) | 3.56 |
| EX-10 | 32.38 | Aluminum | 19.82 (2874) | 9.51 (1379) | 197 |
| EX-11 | 39.09 | Aluminum | 26.42 (3832) | 1161 (168400) | 137 |
| EX-12 | 47.60 | Aluminum | 26.23 (3804) | 1991 (300000) | 2.31 |
| EX-13 | 56.97 | Aluminum | 27.48 (3985) | 2095 (304000) | 2.28 |

| | | | | | |
|---|---|---|---|---|---|
| Examples EX-10 to EX-13 have significantly higher single lap shear strength than CE-3. | | | | | |

**TABLE 8. Summary of the formulations for Examples EX-14 to EX-16**

| | EX-14 | EX-15 | EX-16 |
|---|---|---|---|
| | Mass, g | Mass, g | Mass, g |
| EPON 828 | 18.90 | 18.90 | 18.90 |
| TS720 | 0.59 | 0.59 | 0.59 |
| PE-1 | 1.86 | 3.42 | |
| PE-2 | | | 3.06 |
| TMPMP | 1.99 | 1.99 | 1.99 |
| DMDO | 7.04 | 6.46 | 6.46 |
| K54 | 0.66 | 0.66 | 0.66 |
| 0.005" Beads | 0.19 | 0.19 | 0.19 |
| Total Mass (g) | 31.25 | 32.22 | 31.86 |

**TABLE 9: Overlap shear data to polycarbonate adherends for Comparative Example CE-3 and Examples EX-14 to EX-16.**

| | Substrate | Single lap shear strength, MPa (PSI) |
|---|---|---|
| CE-3 | Polycarbonate | 1.72 (250) |
| EX-14 | Polycarbonate | 4.699 (681.5) |
| EX-15 | Polycarbonate | 2.981 (432.4) |
| EX-16 | Polycarbonate | 3.834 (556.1) |

The cured compositions of EX-14 to EX-16 have significantly higher adhesive strength (e.g., single lap shear strength) over CE-3. Bonding to polycarbonate with conventional thiol-epoxy curable compositions (as in CE-3) is typically difficult.

## Claims

1. A thiol-containing compound having at least two groups of formula -NH-CO-CO-NH-R¹-SH wherein R¹ is an alkylene group.

2. The compound of claim 1, wherein the thiol-containing compound is of Formula (I)
R²-(NH-CO-CO-[NH-R³-NH-CO-CO]ₓ-NH-R¹-SH)ₙ (I)
wherein
R¹ is an alkylene;
R² is an n-valent radical of a (hetero)alkane;
R³ is a (hetero)alkylene;
x is an integer in a range of 0 to 5; and
n is an integer equal to at least 2.

3. The compound of claim 2, wherein n is in a range of 2 to 10.

4. The compound of claim 2 or 3, wherein the variable x is equal to zero and wherein the compound of Formula (I) is of Formula (I-1)
R²-(NH-CO-CO-NH-R¹-SH)ₙ (I-1),
optionally wherein the variable n is equal to 2 or 3.

5. The thiol-containing compound of any one of claims 1 to 4, wherein R¹ is ethylene.

6. A composition comprising a polymerized product of a reaction mixture comprising:
a) a first thiol-containing compound having at least two groups of formula -NH-CO-CO-NH-R¹-SH wherein R¹ is an alkylene; and
b) an epoxy resin.

7. The composition of claim 6, wherein the first thiol-containing compound is of Formula (I)
R²-(NH-CO-CO-[NH-R³-NH-CO-CO]ₓ-NH-R¹-SH)ₙ (I)
wherein
R¹ is an alkylene;
R² is an n-valent radical of a (hetero)alkane;
R³ is a (hetero)alkylene;
x is an integer in a range of 0 to 5; and
n is an integer equal to at least 2.

8. The composition of claim 7, wherein the reaction mixture further comprises a second thiol-containing compound having a plurality of thiol groups and wherein the second thiol-containing compound is not of Formula (I).

9. The composition of claim 7, wherein the first thiol-containing compound of Formula (I) has two groups of formula -NH-CO-CO-NH-R¹-SH and wherein the epoxy resin has two oxirane groups.

10. The composition of claim 9, wherein the reaction mixture further comprises a second thiol-containing compound having two thiol groups and wherein the second thiol-containing compound is not of Formula (I).

11. The composition of claim 9 or 10, wherein the polymerized product is a thermoplastic material.

12. The composition of claim 8, wherein the first thiol-containing compound of Formula (I) has at least three thiol-containing groups of formula -NH-CO-CO-NH-R¹-SH, or the epoxy compound has at least three oxirane groups, or the reaction mixture further comprises a second thiol-containing compound having at least three thiol groups, wherein the second thiol-containing compound is not of Formula (I).

13. The composition of claim 12, wherein the polymerized product is a thermoset material.

14. The composition of any one of claims 6 to 13, further comprising a catalyst.

15. The composition of any one of claims 6 to 14, wherein the composition is an adhesive or sealant.

## Patentansprüche

1. Eine thiolhaltige Verbindung, die mindestens zwei Gruppen von Formel -NH-CO-CO-NH-R¹-SH aufweist, wobei R¹ eine Alkylengruppe ist.

2. Die Verbindung nach Anspruch 1, wobei die thiolhaltige Verbindung aus Formel (I) besteht
R²-(NH-CO-CO-[NH-R³-NH-CO-CO]ₓ-NH-R¹-SH)ₙ (I)
wobei
R¹ ein Alkylen ist;
R² ein n-wertiges Radikal eines (Hetero)alkans ist;
R³ ein (Hetero)alkylen ist;
x eine ganze Zahl in einem Bereich von 0 bis 5 ist; und
n eine ganze Zahl ist, die mindestens gleich 2 ist.

3. Die Verbindung nach Anspruch 2, wobei n in einem Bereich von 2 bis 10 ist.

4. Die Verbindung nach Anspruch 2 oder 3, wobei die Variable x gleich null ist und wobei die Verbindung von Formel (I) aus Formel (I-1) besteht
R²-(NH-CO-CO-NH-R¹-SH)ₙ (1-1),
gegebenenfalls wobei die Variable n gleich 2 oder 3 ist.

5. Die thiolhaltige Verbindung nach einem der Ansprüche 1 bis 4, wobei R¹ Ethylen ist.

6. Eine Zusammensetzung, umfassend ein polymerisiertes Produkt eines Reaktionsgemischs, umfassend:
a) eine erste thiolhaltige Verbindung, die mindestens zwei Gruppen von Formel -NH-CO-CO-NH-R¹-SH aufweist, wobei R¹ ein Alkylen ist; und
b) ein Epoxidharz.

7. Die Zusammensetzung nach Anspruch 6, wobei die erste thiolhaltige Verbindung aus Formel (I) besteht
R²-(NH-CO-CO-[NH-R³-NH-CO-CO]ₓ-NH-R¹-SH)ₙ (I)
wobei
R¹ ein Alkylen ist;
R² ein n-wertiges Radikal eines (Hetero)alkans ist;
R³ ein (Hetero)alkylen ist;
x eine ganze Zahl in einem Bereich von 0 bis 5 ist; und
n eine ganze Zahl ist, die mindestens gleich 2 ist.

8. Die Zusammensetzung nach Anspruch 7, wobei das Reaktionsgemisch ferner eine zweite thiolhaltige Verbindung, die eine Vielzahl von Thiolgruppen aufweist, umfasst und wobei die zweite thiolhaltige Verbindung nicht aus Formel (I) besteht.

9. Die Zusammensetzung nach Anspruch 7, wobei die erste thiolhaltige Verbindung von Formel (I) zwei Gruppen von Formel -NH-CO-CO-NH-R¹-SH aufweist und wobei das Epoxidharz zwei Oxirangruppen aufweist.

10. Die Zusammensetzung nach Anspruch 9, wobei das Reaktionsgemisch ferner eine zweite thiolhaltige Verbindung, die zwei Thiolgruppen aufweist, umfasst und wobei die zweite thiolhaltige Verbindung nicht aus Formel (I) besteht.

11. Die Zusammensetzung nach Anspruch 9 oder 10, wobei das polymerisierte Produkt ein thermoplastisches Material ist.

12. Die Zusammensetzung nach Anspruch 8, wobei die erste thiolhaltige Verbindung von Formel (I) mindestens drei thiolhaltige Gruppen von Formel -NH-CO-CO-NH-R¹-SH aufweist oder die Epoxidverbindung mindestens drei Oxirangruppen aufweist oder das Reaktionsgemisch ferner eine zweite thiolhaltige Verbindung, die mindestens drei Thiolgruppen aufweist, umfasst, wobei die zweite thiolhaltige Verbindung nicht aus Formel (I) besteht.

13. Die Zusammensetzung nach Anspruch 12, wobei das polymerisierte Produkt ein Duroplastmaterial ist.

14. Die Zusammensetzung nach einem der Ansprüche 6 bis 13, ferner umfassend einen Katalysator.

15. Die Zusammensetzung nach einem der Ansprüche 6 bis 14, wobei die Zusammensetzung ein Kleber oder Dichtstoff ist.

## Revendications

1. Composé à teneur en thiol ayant au moins deux groupes de formule -NH-CO-CO-NH-R¹-SH, dans lequel R¹ est un groupe alkylène.

2. Composé selon la revendication 1, dans lequel le composé à teneur en thiol est de Formule (I)
R²-(NH-CO-CO-[NH-R³-NH-CO-CO]ₓ-NH-R¹-SH)ₙ (I)
dans lequel
R¹ est un alkylène ;
R² est un radical de valence n d'un (hétéro)alcane ;
R³ est un (hétéro)alkylène ;
x est un nombre entier compris dans une plage de 0 à 5 ; et
n est un nombre entier égal à au moins 2.

3. Composé selon la revendication 2, dans lequel n est compris dans une plage de 2 à 10.

4. Composé selon la revendication 2 ou 3, dans lequel la variable x est égale à zéro et dans lequel le composé de Formule (I) est de Formule (1-1)
R²-(NH-CO-CO-NH-R¹-SH)ₙ (I-1),
facultativement dans lequel la variable n est égale à 2 ou 3.

5. Composé à teneur en thiol selon l'une quelconque des revendications 1 à 4, dans lequel R¹ est éthylène.

6. Composition comprenant un produit polymérisé d'un mélange réactionnel comprenant :
a) un premier composé à teneur en thiol ayant au moins deux groupes de formule -NH-CO-CO-NH-R¹-SH, dans laquelle R¹ est un alkylène ; et
b) une résine époxy.

7. Composition selon la revendication 6, dans laquelle le composé à teneur en thiol est de Formule (1)
R²-(NH-CO-CO-[NH-R³-NH-CO-CO]ₓ-NH-R¹-SH)ₙ (I)
dans laquelle
R¹ est un alkylène ;
R² est un radical de valence n d'un (hétéro)alcane ;
R³ est un (hétéro)alkylène ;
x est un nombre entier compris dans une plage de 0 à 5 ; et
n est un nombre entier égal à au moins 2.

8. Composition selon la revendication 7, dans laquelle le mélange réactionnel comprend en outre un second composé à teneur en thiol ayant une pluralité de groupes thiol et dans laquelle le second composé à teneur en thiol n'est pas de Formule (I).

9. Composition selon la revendication 7, dans laquelle le premier composé à teneur en thiol de la Formule (I) a deux groupes de formule -NH-CO-CO-NH-R¹-SH et dans laquelle la résine époxy a deux groupes oxirane.

10. Composition selon la revendication 9, dans laquelle le mélange réactionnel comprend en outre un second composé à teneur en thiol ayant deux groupes thiol et dans laquelle le second composé à teneur en thiol n'est pas de Formule (I).

11. Composition selon la revendication 9 ou 10, dans laquelle le produit polymérisé est un matériau thermoplastique.

12. Composition selon la revendication 8, dans laquelle le premier composé à teneur en thiol de Formule (I) a au moins trois groupes à teneur en thiol de formule -NH-CO-CO-NH-R¹-SH, ou le composé époxy a au moins trois groupes oxirane, ou le mélange réactionnel comprend en outre un second composé à teneur en thiol ayant au moins trois groupes thiol, dans laquelle le second composé à teneur en thiol n'est pas de Formule (I).

13. Composition selon la revendication 12, dans laquelle le produit polymérisé est un matériau thermodurci.

14. Composition selon l'une quelconque des revendications 6 à 13, comprenant en outre un catalyseur.

15. Composition selon l'une quelconque des revendications 6 à 14, dans laquelle la composition est un adhésif ou un produit d'étanchéité.
